(19) Europäisches Patentamt
European Patent Office
Office européen des brevets

(11) EP 1 112 734 B1

(12) # FASCICULE DE BREVET EUROPEEN

(45) Date de publication et mention de la délivrance du brevet:
**23.07.2003 Bulletin 2003/30**

(51) Int Cl.[7]: **A61K 7/027**, A61K 7/031, A61K 7/48, A61K 7/025

(21) Numéro de dépôt: **00403334.6**

(22) Date de dépôt: **29.11.2000**

(54) **Composition cosmétique sans transfert comprenant un composé non volatil siliconé et une huile hydrocarbonée non volatile incompatible avec ce composé siliconé**

Abriebfaste kosmetische Zusammensetzung enthaltend eine flüchtige Siliziumverbindung und ein nichtflüchtiges unverträgliches Kohlenwasserstofföl

Non-transfer cosmetic composition comprising a non-volatile silicon compound, and a non-volatile hydrocarbon oil incompatible with the silicone compound

(84) Etats contractants désignés:
**AT BE CH CY DE DK ES FI FR GB GR IE IT LI LU MC NL PT SE TR**

(30) Priorité: **03.12.1999 FR 9915373**

(43) Date de publication de la demande:
**04.07.2001 Bulletin 2001/27**

(73) Titulaire: **L'OREAL**
**75008 Paris (FR)**

(72) Inventeur: **Arnaud, Pascal**
**94240 L'Hay les Roses (FR)**

(74) Mandataire: **Lhoste, Catherine**
**L'OREAL-DPI**
**6 rue Bertrand Sincholle**
**92585 Clichy Cédex (FR)**

(56) Documents cités:
**EP-A- 0 709 083**
**EP-A- 0 748 622**
**EP-A- 0 756 864**
**EP-A- 0 756 865**
**EP-A- 0 819 419**
**EP-A- 0 979 643**
**EP-A- 1 147 761**
**WO-A-96/40044**
**WO-A-97/01321**
**WO-A-97/25960**
**FR-A- 2 765 800**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a trait à une composition contenant un composé non volatil siliconé et une huile hydrocarbonée non volatile incompatibles entre eux, destinée en particulier au domaine cosmétique. Plus spécialement, l'invention se rapporte à une composition sans transfert et brillante pour le soin et/ou le maquillage de la peau aussi bien du visage que du corps humain, des lèvres, des paupières inférieure ou supérieure, ou encore des phanères comme les cils, les sourcils, les ongles et les cheveux.

Cette composition peut se présenter notamment sous forme de produit coulé en stick ou en coupelle comme les rouges ou baumes à lèvres, les fonds de teint coulés, les produits anti-cernes, les fards à paupières ou à joues, sous forme de pâte ou de crème plus ou moins fluide comme les fonds de teint ou rouges à lèvres fluides; les eyes liners, les mascaras, les compositions de protection solaire, de coloration ou de bronzage artificiel de la peau ou encore de maquillage du corps ou des cheveux.

Les produits de maquillage ou de soin de la peau ou des lèvres des êtres humains comme les fonds de teint ou les rouges à lèvres contiennent généralement des phases grasses telles que des cires et des huiles, des pigments et/ou charges et éventuellement des additifs comme des actifs cosmétiques ou dermatologiques. Elles peuvent aussi contenir des produits dits "pâteux", de consistance souple, permettant d'obtenir des pâtes, colorées ou non, à appliquer au pinceau.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, comme notamment un verre, une tasse, une cigarette, un vêtement ou de la peau. Il s'ensuit une persistance médiocre du film appliqué, nécessitant de renouveler régulièrement l'application de la composition de fond de teint ou de rouge à lèvres. Par ailleurs, l'apparition de ces traces inacceptables notamment sur les cols de chemisier peut écarter certaines femmes de l'utilisation de ce type de maquillage.

De plus, ces compositions ont tendance à migrer, c'est-à-dire à se propager à l'intérieur des rides et des ridules de la peau qui entourent les lèvres et les yeux, entraînant un effet inesthétique.

La société Revlon propose, dans sa demande EP-A-0 709 083, une composition ayant des propriétés améliorées de sans transfert, qui comprend des particules de silice triméthylée, un solvant volatil et une huile non volatile. Cependant, cette composition ne présente pas des propriétés cosmétiques, notamment de brillance, totalement satisfaisantes.

La société Procter & Gamble a envisagé dans sa demande de brevet WO-A-96/40044 des compositions de rouge à lèvres présentant des propriétés de sans transfert, contenant une huile volatile et une huile non volatile du type perfluoropolyéther, incompatibles. Dans cette demande, il est, en outre, décrit l'amélioration de la brillance grâce à la dispersion préalable d'une phase huileuse dans une matrice, et la capacité de cette phase huileuse à ségréguer lors de l'application du produit sur le support et à migrer à la surface du film ainsi déposé.

Ce système nécessite cependant une bonne dispersion de la phase huileuse dans la matrice et peut engendrer des problèmes de stabilité du produit liés à la nécessaire mauvaise compatibilité de la phase huileuse avec la matrice.

Il est connu par ailleurs que l'amélioration des propriétés de brillance nécessite une bonne dispersion des particules solides dans la composition en particulier des pigments. Le brevet US 5.945.092 de Revlon décrit ainsi l'utilisation de tensioactifs siliconés associés avec des huiles volatiles.

Malgré leur efficacité, ces tensioactifs présentent l'inconvénient d'être potentiellement irritant notamment pour la muqueuse labiale lorsque leur pourcentage dans la composition est important (typiquement supérieur à 3 %).

Il est donc particulièrement intéressant de trouver un autre moyen d'améliorer la brillance des compositions sans transfert sans avoir les inconvénients cités précédemment.

De plus, ces compositions, bien que présentant des propriétés de "sans transfert" améliorées ont l'inconvénient de laisser sur les lèvres, après évaporation des huiles volatiles, un film qui devient très vite inconfortable au cours du temps (sensation de dessèchement, de tiraillement et d'inconfort), écartant un certain nombre de femmes de ce type de rouge à lèvres. De plus, le dépôt obtenu est mat. Or les consommateurs sont toujours à la recherche d'un produit brillant, confortable à porter tout au long de la journée, qui ne migre pas ou peu dans les plis de la peau entourant les lèvres ou les yeux et qui ne transfère pas ou pratiquement pas.

Il subsiste donc le besoin d'une composition ne présentant pas les inconvénients ci-dessus et ayant notamment de bonnes propriétés de "sans transfert" et de non migration, même lors d'une pression ou d'un frottement prononcé, tout en conférant au dépôt un aspect plus ou moins brillant, adapté au désir de la consommatrice, ne desséchant pas et ne tiraillant pas la peau ou les lèvres sur lesquelles elle est appliquée, aussi bien lors de l'application qu'au cours du temps.

La demanderesse a constaté, de façon tout à fait surprenante, que l'utilisation d'un composé non volatil siliconé, d'une huile hydrocarbonée non volatile, incompatible avec le composé non volatil siliconé, et d'un solvant volatil particulier, dans une composition physiologiquement acceptable et plus spécialement cosmétique, permettait d'obtenir un dépôt brillant, de très bonne tenue, ne transférant peu ou pas du tout, ne migrant pas, résistant à l'eau, tout en

étant très agréable à l'application et à porter tout au long de la journée. Le dépôt est souple et onctueux.

La présente invention a donc pour objet une composition de soin ou de maquillage des matières kératiniques, comprenant au moins un solvant volatil hydrocarboné, au moins un composé non volatil siliconé soluble ou dispersible dans le solvant volatil hydrocarboné et au moins une huile non volatile hydrocarbonée soluble dans le solvant volatil hydrocarboné et incompatible avec le composé non volatil siliconé, l'huile non volatile hydrocarbonée ayant des paramètres de solubilité tels que 16,40 $(J/cm^3)^{1/2} \leq \delta_D \leq$ 19,00 $(J/cm^3)^{1/2}$ et 2,00 $(J/cm^3)^{1/2} \leq \delta_a \leq$ 9,08 $(J/cm^3)^{1/2}$.

Par "huile", on entend tout milieu non aqueux liquide à température ambiante (25°C) et pression atmosphérique (760mm de Hg). Par "solvant", on entend un milieu non aqueux liquide à température ambiante (25°C) et pression atmosphérique (760mm de Hg). Par "volatil", on entend un milieu susceptible de s'évaporer de la peau ou des lèvres, en moins d'une heure. Un milieu volatil est notamment choisi parmi les milieux ayant une pression de vapeur, à température ambiante et pression atmosphérique allant de $10^{-3}$ à 300mm de Hg (0,13 Pa à 40 000 Pa).

Ainsi, la composition peut contenir une ou plusieurs huiles non volatiles hydrocarbonées et un ou plusieurs solvants volatils hydrocarbonés.

Cette composition est en particulier une composition cosmétique ou dermatologique. Elle contient donc des ingrédients compatibles avec la peau, les lèvres, les fibres kératiniques et les ongles. Elle peut se présenter sous forme de gel anhydre, d'émulsion ou de dispersion huile-dans-eau ou eau-dans-huile ou encore sous forme d'émulsion multiple. Elle peut se présenter, en outre, sous forme plus ou moins fluide, de pâte ou de solide non déformable ou rigide, éventuellement coulé en stick ou en coupelle. De préférence, elle se présente sous forme fluide ou de stick en particulier anhydre. Par fluide, on entend une composition s'écoulant sous son propre poids, à l'inverse d'un solide.

Selon l'invention le composé non volatil siliconé et l'huile non volatile hydrocarbonée sont incompatibles entre eux, en l'absence de solvant volatil. Après dépôt de la composition sur les matières kératiniques, le solvant volatil s'évapore, laissant notamment sur ces matières le composé non volatil siliconé, l'huile hydrocarbonée non volatile et, si présente, la charge particulaire. Le dépôt obtenu sur la peau ou les lèvres, après séchage, est homogène, brillant et souple. Il ne laisse pratiquement pas de traces sur un support venant au contact du dépôt et ne migre pas dans les ridules autour des lèvres notamment.

Avantageusement, la composition contient au moins un ingrédient choisi parmi les actifs cosmétiques ou dermatologiques, les matières colorantes et leurs mélanges. Grâce à l'incompatibilité du composé non volatil siliconé et de l'huile hydrocarbonée non volatile, la composition de l'invention permet de limiter, voire supprimer le transfert de la composition et en particulier le transfert des actifs et/ou des matières colorantes et donc de maintenir ces actifs et/ou matières colorantes là où ils ont été déposés.

Un autre objet de l'invention est l'utilisation dans une composition cosmétique ou pour la fabrication d'une composition à application topique de l'association, d'au moins un solvant volatil hydrocarboné, d'au moins un composé non volatil siliconé soluble ou dispersible dans le solvant volatil et d'au moins une huile non volatile hydrocarbonée soluble dans le solvant volatil et incompatible avec le composé non volatil siliconé, l'huile hydrocarbonée non volatile ayant des paramètres de solubilité tels que 16,40 $(J/cm^3)^{1/2} < \delta_D \leq$ 19,00 $(J/cm^3)^{1/2}$ et 2,00 $(J/cm^3)^{1/2} \leq \delta_a \leq$ 9,08 $(J/cm^3)^{1/2}$ comme agent, pour diminuer, voire supprimer, le transfert d'un film de composition déposé sur la peau et/ou les lèvres d'être humain vers un support mis en contact avec le film et/ou pour conserver sa brillance.

Un autre objet de l'invention est l'utilisation dans une composition cosmétique ou pour la fabrication d'une composition à application topique de l'association, d'au moins un solvant volatil hydrocarboné, d'au moins un composé non volatil siliconé soluble ou dispersible dans le solvant volatil et d'au moins une huile non volatile hydrocarbonée soluble dans le solvant volatil et incompatible avec le composé non volatil siliconé, l'huile hydrocarbonée non volatile ayant des paramètres de solubilité tels que 16,40 $(J/cm^3)^{1/2} \leq \delta_D \leq$ 19,00 $(J/cm^3)^{1/2}$ et 2,00 $(J/cm^3)^{1/2} \leq \delta_a \leq$ 9,08 $(J/cm^3)^{1/2}$comme agent, ladite composition étant non-transfert et/ou brillante.

L'invention a encore pour objet un procédé cosmétique de soin ou de maquillage des lèvres, des phanères ou de la peau, consistant à appliquer sur respectivement les lèvres, les phanères ou la peau une composition cosmétique telle définie précédemment.

L'invention a encore pour objet un procédé pour limiter, voire supprimer, le transfert et/ou la migration d'une composition de maquillage ou de soin de la peau ou des lèvres sur un support différent de la dite peau et desdites lèvres, contenant au moins un ingrédient choisi parmi les matières colorantes et les actifs cosmétiques ou dermatologiques, consistant à introduire dans ladite composition un système contenant au moins un composé non volatil siliconé, au moins une huile hydrocarbonée non volatile incompatible avec le composé non volatil siliconé, et au moins une huile volatile solvant du composé non volatil siliconé et de l'huile hydrocarbonée non volatile, tels que définis précédemment.

On a de plus constaté que la composition selon l'invention, présente des qualités d'étalement et d'adhésion sur la peau et les lèvres, particulièrement intéressantes, ainsi qu'un toucher onctueux et agréable. Cette composition a, en outre, l'avantage de se démaquiller facilement notamment avec un lait démaquillant classique. Ceci est tout à fait remarquable puisque les compositions de l'art antérieur à propriétés "sans transfert" élevées sont très difficiles à démaquiller. En général, elles sont vendues avec un produit démaquillant spécifique, ce qui introduit une contrainte

supplémentaire pour l'utilisatrice.

La composition selon l'invention comprend donc avantageusement un ou plusieurs composés non volatils siliconés physiologiquement acceptables.

Les composés non volatils siliconés de l'invention doivent être solubles ou dispersibles dans les solvants volatils hydrocarbonés et notamment dans les isoalcanes volatils. Ils sont choisis parmi les composés liquides à température ambiante et leurs mélanges et de manière encore plus préférentielle, ils ont une viscosité comprise dans la gamme allant de 5 à 10 000 cSt à 25°C et mieux de 10 à 5 000 cSt.

A titre d'exemples on peut citer les polydiméthylsiloxanes, les phényltriméthicones, les polyalkylméthylsiloxanes, les résines de silicones telles celles décrites dans les documents JP-A-62-61911, JP-A-61-65809 et EP-A-602905, les silicones fluorées et leurs mélanges.

En particulier, ces composés siliconés sont choisis parmi les polydiméthylsiloxanes (PDMS) non volatils ; les polydiméthylsiloxanes comportant des groupements alkyle, alcoxy ou phényle, pendant ou en bout de chaîne siliconée, groupements ayant de 2 à 24 atomes de carbone ; les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorés comportant un groupement fluoré pendant ou en bout de chaîne siliconée ayant de 1 à 12 atomes de carbone dont tout ou partie des hydrogène sont substitués par des atomes de fluor ; les résines de silicone et leurs mélanges.

Leur taux massique dans la composition finale est par exemple compris dans la gamme allant de 0,5 à 90 % et de préférence de 5 à 60 % et encore mieux de préférence de 10 à 50 %.

Le composé non volatil siliconé est de préférence en proportion égale ou supérieure à celle de l'huile hydrocarbonée non volatile, autrement dit le rapport R défini par :

$$R = \frac{\text{\% massique composé siliconé non volatil}}{\text{\% massique huile hydrocarbonée non volatil}}$$

est de préférence égal ou supérieur à 1.

Les huiles hydrocarbonées non volatiles de l'invention doivent aussi être solubles dans les solvants volatils hydrocarbonés comme les isoalcanes volatils mais par contre elles ne doivent pas être solubles dans les composés siliconés précédemment décrits.

Ces huiles hydrocarbonées sont fluides à température ambiante et comportent dans leur structure chimique au moins un groupement polaire non ionique suivant :

- COOH
- OH mono ou disubstitué (primaire ou secondaire)
- $PO_4$
- NHR ; $NR_1R_2$, $R_1$ et $R_2$ formant éventuellement un cycle et représentant un radical alkyle ou alcoxy linéaire ou ramifié en $C_1$ à $C_{20}$, ou

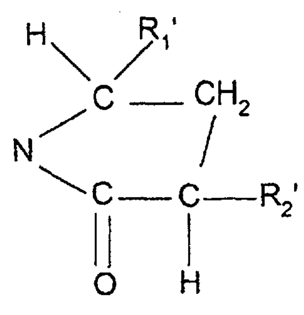

avec $R_1'$ et $R_2'$ qui peuvent être égal à H ou à une chaîne alkyle ou alkoxy linéaire ou ramifiée en $C_1$ à $C_{20}$.

et de préférence au moins deux groupements polaires non ioniques suivants :

- COOH
- OH mono ou disubstitué (primaire ou secondaire)
- $PO_4$

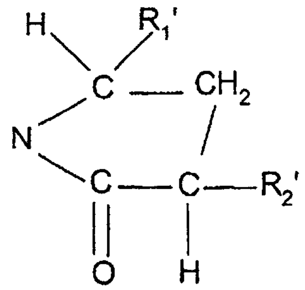

avec $R_1'$ et $R_2'$ qui peuvent être égal à H ou à une chaîne alkyle ou alkoxy linéaire ou ramifiée en $C_1$ à $C_{20}$. De façon avantageuse, les huiles hydrocarbonées comprennent au moins un groupement OH.

De préférence, les huiles hydrocarbonées non volatiles selon l'invention sont telles que leurs paramètres de solubilité de HANSEN, $\delta_D$, $\delta_p$, $\delta_h$ sont tels que : 16,40 $(J/cm^3)^{1/2} \leq \delta_D \leq$ 19,00 $(J/cm^3)^{1/2}$ et de préférence 16,70 $(J/cm^3)^{1/2} \leq \delta_D \leq$ 18,50 $(J/cm^3)^{1/2}$
2,00 $(J/cm^3)^{1/2} \leq \delta_a \leq$ 9,08 $(J/cm^3)^{1/2}$ et de préférence 4,00 $(J/cm^3)^{1/2} \leq \delta_a \leq$ 9,08 $(J/cm^3)^{1/2}$ et encore de préférence 5,00 $(J/cm^3)^{1/2} \leq \delta_a \leq$ 9,08 $(J/cm^3)^{1/2}$ et mieux de 5,00 $(J/cm^3)^{1/2} \leq \delta_a \leq$ 6,80 $(J/cm^3)^{1/2}$
sachant que $\delta_a = (\delta_p^2 + \delta_h^2)^{1/2}$

La définition des paramètres de solubilité selon HANSEN est bien connue de l'homme du métier et notamment décrite dans l'article de C. M. HANSEN : "The three dimensional solubility parameters" J. Paint Technol. 39, 105 (1967).

Selon cet espace de HANSEN :

- $\delta_D$ caractérise les forces de dispersion de LONDON issues de la formation de dipôles induits lors des chocs moléculaires ;
- $\delta_P$ caractérise les forces d'interactions de DEBYE entre dipôles permanents ;
- $\delta_h$ caractérise les forces d'interactions spécifiques (type liaisons hydrogène, acide/base, donneur/accepteur, etc.) ;

Les paramètres $\delta_D$, $\delta_p$ et $\delta_h$ sont généralement exprimés en $(J/cm^3)^{1/2}$.

Dans la composition selon l'invention, on peut utiliser n'importe quelle huile non volatile hydrocarbonée ou mélange d'huiles non volatiles hydrocarbonées satisfaisant aux relations ci-dessus. Dans ce cas, les paramètres de solubilité du mélange sont déterminés à partir de ceux des huiles hydrocarbonées non volatiles prises séparément, selon les relations suivantes :

$$\delta_{Dmel} = \sum_i xi\,\delta_{Di} \quad ; \quad \delta_{p\,mel} = \sum_i xi\,\delta_{pi} \quad \text{et} \quad \delta_{hmel} = \sum_i xi\,\delta_{hi}$$

où xi représente la fraction volumique de l'huile hydrocarbonée non volatile i dans le mélange.

Il est à la portée de l'homme du métier de déterminer les quantités de chaque huile hydrocarbonée non volatile pour obtenir un mélange huiles hydrocarbonées non volatiles satisfaisant aux relations ci-dessus.

La masse moléculaire des huiles hydrocarbonées est, supérieure à 600 g/Mol.

A titre d'exemple, d'huile hydrocarbonée non volatile on peut citer le malate de di-isostéaryle, certains mono ou polyesters de polyols tels que le diisostéarate de diglycéryle, ou le triisostéarate de diglycéryle ou encore des acides poly(hydroxy-12) stéariques tels que le Solsperse 21 000 vendu par la société Zénéca ou l'Arlacel P100 vendu par la société Uniqema, leurs mélanges. De préférence, on utilise le di-isostéarate de diglycéryle, le tri-isostéarate de diglycéryle et les acides poly (hydroxy-12) stéariques et leurs mélanges.

Leur taux massique dans la composition finale, est par exemple compris dans la gamme allant de 2,5 à 40 % et de préférence de 4 à 30 % et mieux de 4 à 15 %.

Comme solvant volatil hydrocarboné utilisable dans l'invention, on peut citer les solvants volatils ayant de 8 à 14 atomes de carbone et leurs mélanges. Ces solvants volatils sont choisis parmi les alcanes ramifiés en $C_8$-$C_{16}$, les esters ramifiés en $C_8$-$C_{16}$ et leurs mélanges. De préférence, ces solvants sont choisis parmi les isoparaffines en $C_8$-$C_{16}$ notamment issues du pétrole, telles que les "Isopars", les "Permetyls", l'isododécane ou l'isohexadécane, le néo-pentanoate d'iso-hexyle et leurs mélanges. De préférence on utilise l'isododécane ou l'isohexadécane ou leurs mélanges.

Le ou les solvants volatils hydrocarbonés selon l'invention, représente notamment un taux massique de 5 à 90 %, de préférence de10 à 60 % et mieux de 20 à 50 %.

La composition peut, en outre, contenir au moins un corps gras additionnel différent du composé non volatil siliconé, du solvant volatil hydrocarboné et de l'huile hydrocarbonée non volatile choisi parmi les cires, les gommes, les corps gras pâteux à température ambiante, les huiles et leurs mélanges, d'origine minérale, animale, végétale ou de synthèse.

Les corps gras huileux additionnels de la composition peuvent être une huile cosmétiquement ou dermatologiquement acceptable, et de façon générale physiologiquement acceptable, notamment choisie parmi les huiles d'origine minérale, animale, végétale ou synthétique, volatiles ou non volatiles.

Comme huiles additionnelles utilisables dans la composition selon l'invention, on peut citer notamment :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame ou de colza, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le parléam
- les esters et les éthers de synthèse notamment d'acides gras comme les huiles de formule $R_3COOR_4$ dans laquelle $R_3$ représente le reste d'un acide gras supérieur comportant de 1 à 40 atomes de carbone et $R_4$ représente une chaîne hydrocarbonée contenant de 1 à 40 atomes de carbone avec $R_3 + R_4 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme le lactate d'isostéaryle, l'hydroxy stéarate d'octyle, l'hydroxy stéarate d'octyl dodécyle, l'adipate de di-isopropyle, le citrate de triisocétyle, des heptanoates, octanoates, décanoates d'alcools gras ; des esters de polyol comme le di-octanoate de propylène glycol, le di-heptanoate de néopentyl glycol, le di-isononanoate de di-éthylèneglycol ; et les esters du pentaérythritol comme le tétra-isostéarate de penta-érythrytyle ;
- des alcools gras ayant de 12 à 26 atomes de carbone comme l'octyl dodécanol, le 2-butyl octanol, le 2-hexyl décanol, le 2-undécylpenta décanol, l'alcool oléique ;
- les huiles fluorées éventuellement volatiles, partiellement hydrocarbonées et/ou siliconées, comme le méthoxynonafluorobutane;
- les huiles siliconées volatiles comme les polydiméthylsiloxanes (PDMS) volatiles, linéaires ou cycliques, ayant de 2 à 7 atomes de silicium, ces silicones comportant éventuellement des groupes alkyle ou alkoxy ayant de 1 à 10 atomes de carbone pendants ou en bout de chaîne siliconée comme l'octaméthyl cyclotétrasiloxane, le décaméthyl cyclopentasiloxane, l'hexadécaméthyl cyclohexasiloxane, l'heptaméthyl hexyltrisiloxane, l'heptaméthyl octyltrisiloxane et leurs mélanges ;
- leurs mélanges.

La ou les huiles non volatiles de la composition peuvent représenter de 0,5 % à 90 % du poids total de la composition et de préférence de 8 à 75 %.

Les huiles volatiles sont favorables à l'obtention d'un film à propriétés "sans transfert". Après évaporation de ces huiles, on obtient un dépôt souple, et homogène, brillant et confortable sur la peau ou les lèvres, sur lesquelles la composition est appliquée. Ces huiles volatiles facilitent, en outre, l'application de la composition sur les matières kératiniques.

Ces huiles volatiles siliconées et fluorées additionnelles représentent notamment de 0 à 30 % du poids total de la composition, et mieux de 0 à 20 %.

La composition de l'invention peut comprendre avantageusement une ou plusieurs matières colorantes contenant au moins (un ou plusieurs) composés pulvérulents et/ou un ou plusieurs colorants liposolubles ou hydrosolubles, par exemple à raison de 0 à 70% du poids total de la composition et notamment de 0,01 à 70%. Le ou les composés pulvérulents peuvent être choisis parmi les pigments, les nacres, les charges habituellement utilisés dans les compositions cosmétiques ou dermatologiques et leurs mélanges. Avantageusement, les composés pulvérulents représentent de 0 à 50 % (par exemple de 0,01 à 50 %) du poids total de la composition et mieux de 1 à 40 %.

Les pigments peuvent être blancs ou colorés, minéraux et/ou organiques, interférentiels ou non, insolubles dans la phase grasse liquide, destinées à colorer et/ou opacifier la composition. On peut citer, parmi les pigments minéraux, le dioxyde de titane, éventuellement traité en surface, les oxydes de zirconium, de zinc ou de cérium, ainsi que les oxydes de zinc, de fer ou de chrome, le violet de manganèse, le bleu outremer, l'hydrate de chrome et le bleu

ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon®(Orgasol® de chez Atochem), de poly-β-alanine et de polyéthylène, les poudres de polytétrafluoroéthylène (Téflon®), la lauroyl-lysine, l'amidon, le nitrure de bore, les microsphères creuses telles que celles de chlorure de polyvinylidène acrylonitrile comme l'Expancel® (Nobel Industrie), les copolymères d'acide acrylique comme le Polytrap® (Dow Corning) et les microbilles de résine de silicone (Tospearl® de Toshiba, par exemple), le carbonate de calcium précipité, le carbonate et l'hydro-carbonate de magnésium, l'hydroxyapatite, les microsphères de silice creuses (Silica Beads® de Maprecos), les microcapsules de verre ou de céramique.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0 à 20 % et notamment 0,01 à 20 % du poids de la compositions et mieux de 0,1 à 6 %. Les colorants hydrosolubles sont par exemple le jus de betterave, le bleu de méthylène et peuvent représenter jusqu'à 6 % du poids total de la composition.

La composition de l'invention peut, en outre, contenir un ou plusieurs actifs cosmétiques ou dermatologiques tels que ceux classiquement utilisés.

Comme actifs cosmétiques ou dermatologiques, utilisables dans la composition de l'invention, on peut citer les hydratants, vitamines, acides gras essentiels, sphingolipides, filtres solaires. Ces actifs sont utilisés en quantité habituelle pour l'homme du métier et notamment à des concentrations de 0 à 20 % et notamment de 0,001 à 20 % du poids total de la composition.

La composition selon l'invention peut, de plus, comprendre, selon le type d'application envisagée, les constituants classiquement utilisés dans les domaines considérés, qui sont présents en une quantité appropriée à la forme galénique souhaitée.

Parmi les cires solides à température ambiante, susceptibles d'être présentes dans la composition selon l'invention, on peut citer les cires hydrocarbonées telles que la cire d'abeilles éventuellement modifiée, la cire de Carnauba, de Candellila, d'Ouricoury, du Japon, les cires de fibres de liège ou de canne à sucre, les cires de paraffine, de lignite, les cires microcristallines, la cire de lanoline, la cire de Montan, les ozokérites, les cires de polyéthylène, les cires obtenues par synthèse de Fischer-Tropsch et les alcools gras en $C_{20}$-$C_{60}$. On peut également utiliser des cires de silicone, parmi lesquelles on peut citer les alkyl, alcoxy et/ou esters de polyméthylsiloxane et leurs mélanges.

Les cires peuvent être présentes à raison de 0-50% (par exemple de 0,01 à 50 %) en poids dans la composition et mieux de 5 à 20 %, en vue de ne pas trop diminuer la brillance de la composition et du film déposé sur les lèvres et/ou la peau.

Comme corps gras pâteux, on peut citer des corps gras ayant un point de fusion allant de 25 à 45 °C et/ou une viscosité à 40 °C allant de 0,1 à 40 Pa.s mesurée au Contraves TV équipé d'un mobile MS-r3 ou Ms-r4 tournant à 60 Hz. A titre d'exemple de corps gras pâteux, on peut citer les PDMS ayant des chaînes pendantes du type alkyle ou alcoxy ayant de 8 à 24 atomes de carbone comme le stearyl diméthicone ; les esters d'alcool gras ou d'acide gras comme les esters du cholestérol, le polylaurate de vinyle, le propionate d'arachidyle ; le copolymère PVP/Eicosène ; les lanolines et leurs dérivés comme les lanolines acétylées ou les lanolines oxypropylènées et leurs mélanges.

La nature et la quantité des cires, des corps gras pâteux et des gommes sont fonction des propriétés mécaniques et de textures recherchées.

La composition peut comprendre, en outre, tout additif usuellement utilisé dans de telles compositions, tel que des épaississants, des antioxydants, des parfums, des conservateurs, des tensioactifs, des polymères liposolubles comme les polyalkylènes, notamment le polybutène, les polyacrylates et les polymères siliconés compatibles avec la phase grasse ainsi que les dérivés de polyvinylpyrolidone. Bien entendu l'homme du métier veillera à choisir ce ou ces éventuels composés complémentaires, et/ou leur quantité, de manière telle que les propriétés avantageuses de la composition selon l'invention ne soient pas ou substantiellement pas, altérées par l'adjonction envisagée.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge ou encore dans une bouillotte. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide. Elles peuvent alors constituer des fonds de teint ou des rouges à lèvres, des brillants à lèvres (gloss en terminologie anglosaxonne), des produits solaires ou de coloration de la peau.

Les compositions de l'invention présentent avantageusement une phase continue grasse (ou lipophile) et sont de préférence anhydres et peuvent contenir moins de 5 % d'eau par rapport au poids total de la composition. Elles peuvent alors se présenter notamment sous forme de gel huileux, de liquide huileux, de pâte ou de stick ou encore sous forme de dispersion vésiculaire contenant des lipides ioniques et/ou non ioniques. Elles peuvent aussi se présenter sous forme d'une émulsion simple ou multiple à phase continue huileuse ou aqueuse, de dispersion huileuse dans une phase aqueuse grâce à des vésicules contenant des lipides ioniques et/ou non ioniques. Ces formes galéniques sont préparées selon les méthodes usuelles des domaines considérés.

De préférence, la composition est un rouge à lèvres comprenant au moins un solvant volatil hydrocarboné, au moins un composé siliconé non volatil, soluble ou dispersible dans le solvant volatil hydrocarboné et au moins une huile non volatile hydrocarbonée soluble dans le solvant volatil et incompatible avec le composé non volatil siliconé, l'huile non volatile hydrocarbonée présentant des paramètres de solubilité tels que 16,40 $(J/cm^3)^{1/2} \leq \delta_D \leq$ 19,00 $(J/cm^3)^{1/2}$et 2,00 $(J/cm^3)^{1/2} \leq \delta_a \leq$ 9,08 $(J/cm^3)^{1/2}$, le solvant, le composé siliconé et l'huile hydrocarbonée étant tels que définis précédemment.

Ces compositions à application topique peuvent constituer notamment une composition cosmétique ou dermatologique, de protection, de traitement ou de soin pour le visage, pour le cou, pour les mains ou pour le corps (par exemple crème de soin, huile solaire, gel corporel), une composition de maquillage (par exemple gel de maquillage, crème, stick)
ou une composition de bronzage artificiel ou de protection de la peau.

L'invention est illustrée plus en détail dans les exemples suivants. Les pourcentages sont des pourcentages en poids.

**Exemples 1 et 2 : Rouges à lèvres sous forme de « gloss » en bouillotte.**

Nous avons comparé les formules suivantes :

| *Phase A* | *Exemple 1* | *Exemple 2* |
|---|---|---|
| . polyisobutène hydrogéné | 11,15 % | 11,15 % |
| . acide poly(hydroxy-12) stéarique vendu sous la référence Solsperse 21000 par la société Zénéca | 4,86 % | - |
| . conservateur | qsp | qsp |
| . antioxydant | qsp | qsp |
| *Phase B* | | |
| . cire microcristalline | 7,63 % | 7,63 % |
| . ozokérite | 5,27 % | 5,27 % |
| *Phase C* | | |
| . oxyde de titane | 0,38 % | 0,38 % |
| . DC Red n°7 | 1,38 % | 1,38 % |
| . FDC yellow n°6 Al lake | 2,94 % | 2,94 % |
| . kaolin | 7,50 % | 7,50 % |
| *Phase D* | | |
| . phényltriméthicone (1000 cSt) | 36,35 % | 41,21 % |
| *Phase E* | | |
| . isododécane | 22,08 %<br>100 % massique | 22,08 %<br>100 % massique |

Mode opératoire

La phase particulaire C est broyée dans la phase A à l'aide d'un broyeur tricylindre. On ajoute ensuite la phase cireuse B et l'on chauffe à 100°C jusqu'à dissolution complète des cires. On ajoute la phényltriméthicone à 100°C puis l'isododécane à 80-90°C. Après homogénéisation on peut introduire la composition à 60-80°C dans des bouillotes adéquates. L'exemple n°1 a des propriétés de brillance et de non transfert supérieures à celles de l'exemple n°2.

**Exemple n°3 : Rouge à lèvres sous forme de « gloss » en bouillotte**

*Phase A*

- polyisobutène hydrogéné 11,15 %
- disostéarate de diglycéryle vendu sous la référence 4,86 % Salacos 42 par la société Nisshin Oil Mills
- conservateur qsp
- antioxydant qsp

*Phase B*

- cire microcristalline 7,63 %
- ozokérite 5,27 %

*Phase C*

- oxyde de titane 0,38 %
- DC Red n°7 1,38 %
- FDC yellow n°6 Al lake 2,94 %
- kaolin 7,50 %

*Phase D*

- phényltriméthicone (1000 cSt) 36,35 %

*Phase E*

- isododécane 22,08 % 100 % massique

**Exemple n°4 : Rouge à lèvres sous forme de « gloss » en bouillotte**

*Phase A*

- polyisobutène hydrogéné 10,95 %
- acide poly(hydroxy-12) stéarique vendu sous la référence Solsperse 21000 par la société Zénéca 4,86 %
- alpha bisabolol 0,2 %
- PVP/Eicosène copolymer 5,55 %
- conservateur qsp

- antioxydant qsp

*Phase B*

- cire microcristalline 4,35 %
- ozokérite 3,00 %

*Phase C*

- oxyde de titane 0,28 %
- DC Red n°7 1,03 %
- FDC yellow n°6 Al lake 2,19 %
- kaolin 7,00 %
- acrylates copolymer (Polytrap®) 0,5 %

*Phase D*

- phényltriméthicone (1000 cSt) 36,35 %

*Phase E*

- mica 1,2 %

*Phase F*

- isododécane 22,08 % 100 % massique

**Exemple 5 : Rouge à lèvres en stick**

*Phase A*

- polyisobutène hydrogéné 11,00 %
- disostéarate de diglycéryle vendu sous la référence Salacos 42 par la société Nisshin Oil Mills 4,86 %
- PVP/Eicosène copolymer 5,00 %
- antioxydant qsp

*Phase B*

- cire de polyéthylène (Mn* = 400) 10,00 %

*Phase C*

- oxyde de titane 0,28 %
- DC Red n°7 1,03 %
- FDC yellow n°6 Al lake 2,19 %
- kaolin 7,50 %

*Phase D*

- phényltriméthicone (1000 cSt) 35,00 %

*Phase E*

- mica 1,00 %

*Phase F*

- isododécane 22,08 % 100 % massique

* Mn = masse moléculaire moyenne en nombre.

Mode opératoire

La phase particulaire C est broyée dans la phase A à l'aide d'un broyeur tricylindre. On ajoute ensuite la cire de polyéthylène et l'on chauffe à 100°C jusqu'à dissolution complète de la cire. On ajoute ensuite la phényltriméthicone et le mica à 100°C, puis l'isododécane à 80-90°C. On peut ensuite couler à 80-85°C dans des moules adéquats pour obtenir des sticks.

La composition de l'exemple 4 a été testée en comparaison avec une formule commerciale de rouge à lèvres liquide « Liquid Lip » de REVLON ne contenant pas l'association selon l'invention de composé siliconé non volatil, de solvant volatil hydrocarboné et d'huile non volatile hydrocarbonée.

La composition de l'invention a été jugée d'aspect plus fluide et plus brillante que celle de l'art antérieur.

La composition de l'invention a été jugée plus brillante lors de l'application et dans le temps ainsi que beaucoup plus confortable.

A l'application, les deux rouges à lèvres ne collent pas. La tenue dans le temps a été jugée identique ; ils transfèrent peu, sont tenaces nécessitant ainsi un démaquillage. Ils s'estompent uniformément dans le temps.

**Exemple 6 : Rouge à lèvres en stick**

*Phase A*

- polyisobutène hydrogéné 14,36 %
- acide polyhydroxy-12 stéarique vendu sous la référence Solsperse 21000 par la société Zeneca 4,00 %
- PVP/Eicosène copolymer 5,00 %
- alpha bisabobol 0,40 %
- antioxydant qsp

*Phase B*

- cire de carnauba 2,02 %
- ozokérite 2,40 %
- cire de polyéthylène (Mn* = 650) 8,08 %

*Phase C*

- oxyde de titane 1,80 %
- DC Red n°7 ca lake 2,90 %
- FDC yellow n°6 Al lake 3,30 %
- DC Red n°21 Al lake 0,60 %
- oxyde de fer noir 0,06 %
- kaolin 1,84 %

*Phase D*

- phényltriméthicone (1000 cSt) 34,32 %

*Phase E*

- mica 1,14 %

*Phase F*

- isododécane 17,72 % 100 % massique

* Mn = masse moléculaire moyenne en nombre.

<u>Mode opératoire</u>

La phase particulaire C est broyée dans la phase A à l'aide d'un broyeur tricylindre. On ajoute ensuite la phase cireuse B et l'on chauffe à 105°C jusqu'à dissolution complète des cires. On ajoute ensuite la phényltriméthicone et le mica à 105°C, puis l'isododécane à 90-100°C. On peut ensuite couler à 90-100°C dans des moules adéquats pour obtenir des sticks de bonne tenue, non collant, ne transférant pas, agréable à porter.

**Exemple 7 : Rouge à lèvres en stick**

*Phase A*

- polyisobutène hydrogéné 13,82 %
- triisostéarate de diglycéryle vendu sous la référence Salacos 43 par la société Nisshin Oil Mills 3,85 %
- PVP/Eicosène copolymer 4,82 %
- alpha bisabolol 0,40 %
- antioxydant qsp

*Phase B*

- ozokérite 3,40 %
- cire de polyéthylène (Mn* = 500) 11,60 %

*Phase C*

- DC Red n°7 2,72 %
- FDC Blue n°1 Al lake 0,81 %
- oxyde de fer brun 1,01 %
- oxyde de fer noir 2,01 %
- kaolin 3,94 %

*Phase D*

- phényltriméthicone (1000 cSt) 24,11 %

*Phase E*

- nacres 5,45 %

*Phase F*

- isododécane 22,00 % 100 % massique

* Mn = masse moléculaire moyenne en nombre.

Le mode opératoire est identique à celui de l'exemple 6. Les propriétés cosmétiques du stick sont identiques à celles du stick de l'exemple 6.

**Exemple 8 : Rouge à lèvres en stick**

*Phase A*

- acide polyhydroxy-12 stearique vendu sous la référence Solsperse 21000 par la société Zeneca 4,00 %
- PVP/Eicosène copolymer 3,00 %
- Propionate d'arachidyle 5,00 %
- $\alpha$ bisabolol 0,40 %
- antioxydant qsp

*Phase B*

- polyisobutène hydrogéné 18,76 %
- bentone 0,54 %

*-Phase C*

- ozokérite 3,78 %
- cire de polyéthylène (Mn* = 650) 11,22 %

*Phase D*

- oxyde de titane 1,80 %
- DC Red n°7 ca lake 2,90 %
- FDC yellow n°6 Al lake 3,30 %
- DC Red n°21 Al lake 0,60 %
- oxyde de fer noir 0,06 %
- kaolin 1,84 %

*Phase E*

- phényltriméthicone (1000 cSt) 21,60 %

*Phase F*

- mica 1,14 %

*Phase F*

- isohexadécane 20,00 % 100 % massique

* Mn = masse moléculaire moyenne en nombre.

Mode opératoire

La phase B est réalisée en dispersant la bentone dans le polyisobutène hydrogéné. La phase particulaire D est broyée dans la phase A à l'aide d'un broyeur tricylindre. On ajoute ensuite la phase B puis la phase cireuse C. On chauffe à 105°C jusqu'à dissolution complète des cires. On ajoute ensuite la phényltriméthicone et le mica à 105°C, puis l'isohexadécane à 90-100°C. On peut ensuite couler la composition à 90-100°C dans des moules adéquats pour obtenir des sticks ayant des propriétés identiques au stick de l'exemple 6.

## Revendications

**1.** Composition de soin ou de maquillage des matières kératiniques, comprenant au moins un solvant volatil hydrocarboné choisi parmi les alcanes ramifiés en $C_8$-$C_{16}$, les esters ramifiés en $C_8$-$C_{14}$ et leurs mélanges, au moins un composé siliconé non volatil liquide à température ambiante, soluble ou dispersible dans le solvant volatil hydrocarboné et au moins une huile non volatile hydrocarbonée soluble dans le solvant volatil et incompatible avec le composé non volatil siliconé, l'huile non volatile hydrocarbonée présentant des paramètres de solubilité tels que 16,40 $(J/cm^3)^{1/2} \leq \delta_D \leq$ 19,00 $(J/cm^3)^{1/2}$ et 2,00 $(J/cm^3)^{1/2} \leq \delta_a \leq$ 9,08 $(J/cm^3)^{1/2}$ et une masse molaire supérieure à 600 g/mol.

**2.** Composition selon la revendication 1, **caractérisée en ce que** l'huile hydrocarbonée présente des paramètres de

solubilité tels que 16,70 $(J/cm^3)^{1/2} \leq \delta_D \leq 18{,}50\ (J/cm^3)^{1/2}$.

3. Composition selon la revendication 2, **caractérisée en ce que** l'huile hydrocarbonée présente des paramètres de solubilité tels que 4,00 $(J/cm^3)^{1/2} \leq \delta_a \leq 9{,}08\ (J/cm^3)^{1/2}$.

4. Composition selon l'une des revendications précédentes, **caractérisée en ce que** l'huile hydrocarbonée présente une structure chimique comportant au moins un groupement polaire non ionique choisi parmi -COOH ; -OH ; $-PO_4$ ; NHR ; $NR_1R_2$ avec $R_1$, $R_2$ formant éventuellement un cycle et représentant un radical alkyle ou alkoxy linéaire ou ramifié en Ci à $C_{20}$ ou

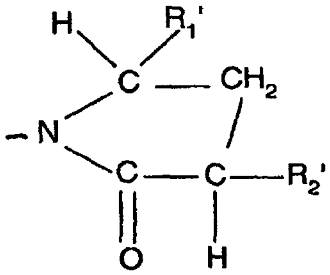

avec $R_1$' et $R_2$' qui peuvent être égal à H ou à une chaîne alkyle ou alkoxy linéaire ou ramifiée en $C_1$ à $C_{20}$.

5. Composition selon l'une des revendications précédentes, **caractérisée en ce que** l'huile hydrocarbonée présente une structure chimique comportant au moins deux groupements polaire non ionique choisis parmi -COOH ; -OH ; $-PO_4$ ; ou

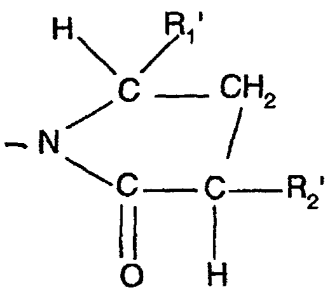

avec $R_1$' et $R_2$' qui peuvent être égal à H ou à une chaîne alkyle ou alkoxy linéaire ou ramifiée en $C_1$ à $C_{20}$.

6. Composition selon l'une des revendications précédentes, **caractérisée en ce que** l'huile hydrocarbonée non volatile est choisie parmi le malate di-isostéaryle, les mono et polyesters de polyol, les acides poly(hydroxy-12) stéariques et leurs mélanges.

7. Composition selon l'une des revendications précédentes, **caractérisée en ce que** l'huile hydrocarbonée non volatile est choisie parmi le di-isostéarate de diglycéryle, le tri-isostéarate de diglycéryle, les acides poly(hydroxy-12) stéariques et leurs mélanges.

8. Composition selon l'une des revendications précédentes, **caractérisée en ce que** l'huile hydrocarbonée non volatile est présente en une teneur massique allant de 2,5 à 40 % et de préférence de 4 à 30 % et mieux de 4 à 15 %.

9. Composition selon l'une des revendications précédentes, dans laquelle le composé non volatil siliconé présente une viscosité choisie dans la gamme allant de 5 à 10 000 cSt et de préférence allant de 10 à 5 000 cSt (0.1-50 $cm^2/s$).

10. Composition selon l'une des revendications précédentes, dans laquelle le composé non volatil siliconé est choisi parmi les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone ;

les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les silicones fluorés comportant un groupement fluoré pendant ou en bout de chaîne siliconée ayant de 1 à 12 atomes de carbone dont tout ou partie des hydrogène sont substitués par des atomes de fluor ; les résines de silicone et leurs mélanges.

**11.** Composition selon l'une des revendications précédentes, **caractérisée en ce que** le composé non volatil siliconé représente un taux massique de 0,5 à 90 %, de préférence de 5 à 60 % et mieux de 10 à 50 %.

**12.** Composition selon l'une des revendications précédentes, dans laquelle au moins un ingrédient choisi parmi les actifs cosmétiques, les actifs dermatologiques et les matières colorantes est prévu.

**13.** Composition selon l'une des revendications précédentes, dans laquelle le solvant volatil est choisi parmi les isoparaffines en $C_8$-$C_{16}$, l'isododécane, l'isohexadécane et leurs mélanges.

**14.** Composition selon l'une des revendications précédentes, dans la laquelle le solvant volatil est choisi parmi l'isododécane, l'isohexadécane et leurs mélanges.

**15.** Composition selon l'une des revendications précédentes, dans laquelle le solvant volatil représente un taux massique de 5 à 90 %, de préférence de 10 à 60 % et mieux de 20 à 50 %.

**16.** Composition selon l'une revendications précédentes, dans laquelle le rapport massique de composé siliconé non volatil par rapport à l'huile hydrocarbonée non volatile est égal ou supérieur à 1.

**17.** Composition selon l'une des revendications précédentes, comprenant, en outre, au moins un corps gras différent du composé non volatil siliconé, du solvant volatil hydrocarboné et de l'huile hydrocarbonée non volatile choisi parmi les cires, les gommes, les corps gras pâteux à température ambiante, les huiles et leurs mélanges.

**18.** Composition selon la revendication 12, dans laquelle les matières colorantes comprennent au moins un composé pulvérulent choisi parmi les charges, les pigments, les nacres et leurs mélanges.

**19.** Composition selon la revendication 18, **caractérisée en ce que** le composé pulvérulent représente jusqu'à 50 % du poids total de la composition.

**20.** Composition selon l'une des revendications précédentes, se présentant sous forme d'un stick ou bâton, sous la forme d'une pâte souple, sous forme de coupelle, de gel huileux, de liquide huileux, de dispersion vésiculaire contenant des lipides ioniques et/ou non ioniques, d'émulsion eau-dans-huile ou huile-dans-eau.

**21.** Composition selon l'une des revendications précédentes, se présentant sous forme anhydre.

**22.** Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

**23.** Rouge à lèvres comprenant au moins un solvant volatil hydrocarboné choisi parmi les alcanes ramifiés en $C_8$-$C_{16}$, les esters ramifiés en $C_8$-$C_{14}$ et leurs mélanges, au moins un composé siliconé non volatil liquide à température ambiante, soluble ou dispersible dans le solvant volatil hydrocarboné et au moins une huile non volatile hydrocarbonée soluble dans le solvant volatil et incompatible avec le composé non volatil siliconé, l'huile non volatile hydrocarbonée présentant des paramètres de solubilité tels que $16{,}40\ (J/cm^3)^{1/2} \leq \delta_D \leq 19{,}00\ (J/cm^3)^{1/2}$ et $2{,}00\ (J/cm^3)^{1/2} \leq \delta_a \leq 9{,}08\ (J/cm^3)^{1/2}$ et une masse molaire supérieure à 600 g/mol.

**24.** Utilisation dans une composition cosmétique ou pour la fabrication d'une composition à application topique, de l'association d'au moins un solvant volatil hydrocarboné choisi parmi les alcanes ramifiés en $C_8$-$C_{16}$, les esters ramifiés en $C_8$-$C_{14}$ et leurs mélanges, d'au moins un composé non volatil siliconé liquide à température ambiante, soluble ou dispersible dans le solvant volatil et d'au moins une huile non volatile hydrocarbonée soluble dans le solvant volatil et incompatible avec le composé non volatil siliconé, l'huile hydrocarbonée non volatile présentant des paramètres de solubilité tels que $16{,}40\ (J/cm^3)^{1/2} \leq \delta_D \leq 19{,}00\ (J/cm^3)^{1/2}$ et $2{,}00\ (J/cm^3)^{1/2} \leq \delta_a \leq 9{,}08\ (J/cm^3)^{1/2}$ et une masse molaire supérieure à 600 g/mol, comme agent pour diminuer, voire supprimer, le transfert

d'un film de composition déposé sur la peau et/ou les lèvres d'être humain vers un support mis en contact avec le film et/ou pour conserver sa brillance.

**25.** Utilisation dans une composition cosmétique ou pour la fabrication d'une composition à application topique de l'association, d'au moins un solvant volatil hydrocarboné choisi parmi les alcanes ramifiés en $C_8$-$C_{16}$, les esters ramifiés en $C_8$-$C_{14}$ et leurs mélanges, d'au moins un composé non volatil siliconé liquide à température ambiante, soluble ou dispersible dans le solvant volatil et d'au moins une huile non volatile hydrocarbonée soluble dans le solvant volatil et incompatible avec le composé non volatil siliconé, l'huile hydrocarbonée non volatile ayant des paramètres de solubilité tels que 16,40 $(J/cm^3)^{1/2} \leq \delta_D \leq$ 19,00 $(J/cm^3)^{1/2}$ et 2,00 $(J/cm^3)^{1/2} \leq \delta_a \leq$ 9,08 $(J/cm^3)^{1/2}$ et une masse molaire supérieure à 600 g/mol, ladite composition étant non transfert et/ou brillant.

**26.** Procédé non-thérapeutique de soin cosmétique ou de maquillage des lèvres ou de la peau, consistant à appliquer sur respectivement les lèvres ou la peau une composition cosmétique telle définie aux revendications 1 à 24.

**27.** Procédé cosmétique pour limiter, voire supprimer, le transfert d'une composition de maquillage ou de soin cosmétique de la peau ou des lèvres sur un support différent de la peau ou des lèvres, contenant au moins un ingrédient choisi parmi les actifs cosmétiques et les matières colorantes, consistant à introduire dans la composition un système contenant au moins un solvant volatil hydrocarboné choisi parmi les alcanes ramifiés en $C_8$-$C_{16}$, les esters ramifiés en $C_8$-$C_{14}$ et leurs mélanges, au moins un composé non volatil siliconé liquide à température ambiante, soluble ou dispersible dans le solvant volatil et au moins une huile non volatile hydrocarbonée soluble dans le solvant volatil et incompatible avec le composé non volatil siliconé, l'huile hydrocarbonée présentant des paramètres de solubilité tels que 16,40 $(J/cm^3)^{1/2} \leq \delta_D \leq$ 19,00 $(J/cm^3)^{1/2}$ et 2,00 $(J/cm^3)^{1/2} \leq \delta_a \leq$ 9,08 $(J/cm^3)^{1/2}$ et une masse molaire supérieure à 600 g/mol.

## Patentansprüche

**1.** Zusammensetzung zur Pflege oder zum Schminken von Keratinsubstanzen, die mindestens einen als Lösungsmittel dienenden, flüchtigen Kohlenwasserstoff, der unter den verzweigten $C_{8-16}$-Alkanen, den verzweigten $C_{8-14}$-Estern und deren Gemischen ausgewählt ist, mindestens ein bei Raumtemperatur flüssiges, in dem flüchtigen Kohlenwasserstoff lösliches oder dispergierbares, nicht flüchtiges Silicon und mindestens ein in dem-flüchtigen Lösungsmittel lösliches und mit dem nicht flüchtigen Silicon inkompatibles, nicht flüchtiges Kohlenwasserstofföl enthält, wobei das nicht flüchtige Kohlenwasserstofföl die Löslichkeitsparameter 16,40 $(J/cm^3)^{1/2} \leq \delta_D \leq$ 19,00 $(J/cm^3)^{1/2}$ und 2,00 $(J/cm^3)^{1/2} \leq \delta_a \leq$ 9,08 $(J/cm^3)^{1/2}$ aufweist und eine Molmasse über 600 g/mol besitzt.

**2.** Zusammensetzung nach Anspruch 1, **dadurch gekennzeichnet, dass** das Kohlenwasserstofföl die Löslichkeitsparameter 16,70 $(J/cm^3)^{1/2} \leq \delta_D \leq$ 18,50 $(J/cm^3)^{1/2}$ aufweist.

**3.** Zusammensetzung nach Anspruch 2, **dadurch gekennzeichnet, dass** das Kohlenwasserstofföl die Löslichkeitsparameter 4,00 $(J/cm^3)^{1/2} \leq \delta_a \leq$ 9,08 $(J/cm^3)^{1/2}$ aufweist.

**4.** Zusammensetzung nach einem der vorhergehenden Ansprüche, **dadurch gekennzeichnet, dass** das Kohlenwasserstofföl eine chemische Struktur aufweist, die mindestens eine nichtionische, polare Gruppe enthält, die ausgewählt ist unter -COOH; -OH; $-PO_4$; NHR; $NR_1R_2$, wobei $R_1$ und $R_2$, die gegebenenfalls einen Ring bilden können, eine geradkettige oder verzweigte Alkyl- oder Alkoxygruppe mit 1 bis 20 Kohlenstoffatomen bedeuten, oder

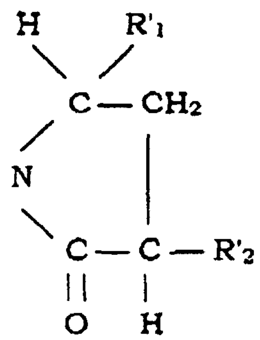

wobei $R_1$' und $R_2$' H oder eine geradkettige oder verzweigte Alkyloder Alkoxygruppe mit 1 bis 20 Kohlenstoffatomen bedeuten können.

5. Zusammensetzung nach einem der vorhergehenden Ansprüche, **dadurch gekennzeichnet, dass** das Kohlenwasserstofföl eine chemische Struktur besitzt, die mindestens zwei nichtionische polare Gruppen aufweist, die ausgewählt sind unter -COOH; -OH; -$PO_4$; oder

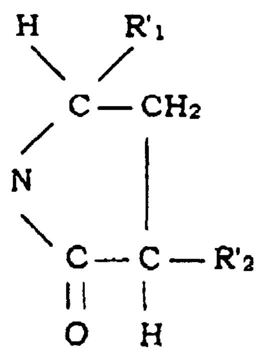

wobei $R_1$' und $R_2$' H oder eine geradkettige oder verzweigte Alkyloder Alkoxygruppe mit 1 bis 20 Kohlenstoffatomen bedeuten können.

6. Zusammensetzung nach einem der vorhergehenden Ansprüche, **dadurch gekennzeichnet, dass** das nicht flüchtige Kohlenwasserstofföl unter Diisostearylmalat, Mono- und Polyestern von Polyolen, Poly(12-hydroxy)stearinsäuren und deren Gemischen ausgewählt ist.

7. Zusammensetzung nach einem der vorhergehenden Ansprüche, **dadurch gekennzeichnet, dass** das nicht flüchtige Kohlenwasserstofföl unter Diglyceryldiisostearat, Diglyceryltriisostearat, Poly(12-hydroxy)stearinsäuren und deren Gemischen ausgewählt ist.

8. Zusammensetzung nach einem der vorhergehenden Ansprüche, **dadurch gekennzeichnet, dass** das nicht flüchtigen Kohlenwasserstöfföl in einem Gewichtsanteil von 2,5 bis 40 %, vorzugsweise 4 bis 30 % und besser 4 bis 15 % vorliegt.

9. Zusammensetzung nach einem der vorhergehenden Ansprüche, wobei das nicht flüchtige Silicon eine Viskosität im Bereich von 5 bis 10 000 cSt und vorzugsweise 10 bis 5 000 cSt (0,1 bis 50 $cm^2/s$) aufweist.

10. Zusammensetzung nach einem der vorhergehenden Ansprüche, **dadurch gekennzeichnet, dass** das nicht flüchtige Silicon unter den nicht flüchtigen Polydimethylsiloxanen (PDMS); Polydimethylsiloxanen, die Alkyl-, Alkoxyoder Phenylgruppen mit 2 bis 24 Kohlenstoffatomen als Seitengruppen oder am Ende der Siliconkette aufweisen; Phenyltrimethiconen, Phenyldimethiconen, Phenyltrimethylsiloxydiphenylsiloxanen, Diphenyldimethiconen, Diphenylmethyldiphenyltrisiloxanen, 2-Phenylethyltrimethylsiloxysilicaten; fluorierten Siliconen, die als Seitengruppe oder am Ende der Siliconkette eine fluorierte Gruppe mit 1 bis 12 Kohlenstoffatomen aufweisen, deren Wasserstoffatome ganz oder teilweise durch Fluoratome ersetzt sind; Siliconharzen und deren Gemischen ausgewählt ist.

**11.** Zusammensetzung nach einem der vorhergehenden Ansprüche, **dadurch gekennzeichnet, dass** das nicht flüchtige Silicon in einem Gewichtsanteil von 0,5 bis 90 %, vorzugsweise 5 bis 60 % und besser 10 bis 50 % vorliegt.

**12.** Zusammensetzung nach einem der vorhergehenden Ansprüche, wobei mindestens ein Bestandteil enthalten ist, der unter den kosmetischen Wirkstoffen, dermatologischen Wirkstoffen und Farbmitteln ausgewählt ist.

**13.** Zusammensetzung nach einem der vorhergehenden Ansprüche, wobei das flüchtige Lösungsmittel unter den $C_{8-16}$-Isoparaffinen, Isododecan, Isohexadecan und deren Gemischen ausgewählt ist.

**14.** Zusammensetzung nach einem der vorhergehenden Ansprüche, wobei das flüchtige Lösungsmittel unter Isododecan, Isohexadecan und deren Gemischen ausgewählt ist.

**15.** Zusammensetzung nach einem der vorhergehenden Ansprüche, wobei das flüchtige Lösungsmittel in einem Massegehalt von 5 bis 90 %, vorzugsweise 10 bis 60 % und besser 20 bis 50 % vorliegt.

**16.** Zusammensetzung nach einem der vorhergehenden Ansprüche, wobei das Masseverhältnis von nicht flüchtigem Silicon und nicht flüchtigem kohlenwasserstoffhaltigen Öl 1 beträgt oder darüber liegt.

**17.** Zusammensetzung nach einem der vorhergehenden Ansprüche, die ferner mindestens eine Fettsubstanz enthält, die von dem nicht flüchtigen Silicon, dem flüchtigen Kohlenwasserstoff und dem nicht flüchtigen Kohlenwasserstofföl verschieden und unter den Wachsen, Gummis/Gummen, bei Raumtemperatur pastösen Fettsubstanzen, Ölen und deren Gemischen ausgewählt ist.

**18.** Zusammensetzung nach Anspruch 12, wobei die Farbmittel mindestens eine pulverförmige Verbindung umfassen, die unter den Füllstoffen, Pigmenten, Perlglanzpigmenten und deren Gemischen ausgewählt ist.

**19.** Zusammensetzung nach Anspruch 18, **dadurch gekennzeichnet, dass** die pulverförmige Verbindung bis zu 50 % des Gesamtgewichts der Zusammensetzung ausmacht.

**20.** Zusammensetzung nach einem der vorhergehenden Ansprüche, die als Stick oder Stift, in Form einer weichen Paste, in Form eines Tiegelchens oder als öliges Gel, ölige Flüssigkeit, Vesikeldispersion, die ionische und/oder nichtionische Lipide enthält, Wasser-in-Öl-Emulsion oder Öl-in-Wasser-Emulsion vorliegt.

**21.** Zusammensetzung nach einem der vorhergehenden Ansprüche, die wasserfrei ist.

**22.** Zusammensetzung nach einem der vorhergehenden Ansprüche, die als Make-up, Wagenrouge, Lidschatten, Lippenstift, Pflegebasis oder Pflegebalsam für die Lippen, Produkt gegen Augenringe, Eyeliner oder Mascara vorliegt.

**23.** Lippenstift, der mindestens einen flüchtigen Kohlenwasserstoff als Lösungsmittel, der unter den verzweigten $C_{8-16}$-Alkanen, den verzweigten $C_{8-14}$-Estern und deren Gemischen ausgewählt ist, mindestens ein bei Raumtemperatur flüssiges, in dem flüchtigen Kohlenwasserstoff lösliches oder dispergierbares, nicht flüchtiges Silicon und mindestens ein in dem flüchtigen Lösungsmittel lösliches und mit dem nicht flüchtigen Silicon inkompatibles, nicht flüchtiges Kohlenwasserstofföl enthält, wobei das nicht flüchtige Kohlenwasserstofföl die Löslichkeitsparameter 6,40 $(J/cm^3)^{1/2} \leq \delta_D \leq 19{,}00\ (J/cm^3)^{1/2}$ und 2,00 $(J/cm^3)^{1/2} \leq \delta_a \leq 9{,}08\ (J/cm^3)^{1/2}$ aufweist und eine Molmasse über 600 g/mol besitzt.

**24.** Verwendung der Kombination mindestens eines als Lösungsmittel dienenden flüchtigen Kohlenwasserstoffs, der unter den verzweigten $C_{8-16}$-Alkanen, verzweigten $C_{8-14}$-Estern und deren Gemischen ausgewählt ist, mindestens eines bei Raumtemperatur flüssigen, in dem flüchtigen Kohlenwasserstoff löslichen oder dispergierbaren, nicht flüchtigen Silicons und mindestens eines in dem flüchtigen Lösungsmittel löslichen und mit dem nicht flüchtigen Silicon inkompatiblen, nicht flüchtigen Kohlenwasserstofföls, wobei das nicht flüchtige Kohlenwasserstofföl die Löslichkeitsparameter 16,40 $(J/cm^3)^{1/2} \leq \delta_D \leq 19{,}00\ (J/cm^3)^{1/2}$ und 2,00 $(J/cm^3)^{1/2} \leq \delta_a \leq 9{,}08\ (J/cm^3)^{1/2}$ und eine Molmasse über 600 g/mol aufweist, in einer kosmetischen Zusammensetzung oder zur Herstellung einer Zusammensetzung zur topischen Anwendung als Mittel, um den Transfer eines Films der auf der menschlichen Haut und/oder den menschlichen Lippen abgeschiedenen Zusammensetzung auf einen mit dem Film in Kontakt stehenden Träger zu vermindern oder sogar zu verhindern und/oder seinen Glanz zu bewahren.

**25.** Verwendung der Kombination mindestens eines als Lösungsmittel dienenden, flüchtigen Kohlenwasserstoffs, der unter den verzweigten $C_{8-16}$-Alkanen, verzweigten $C_{8-14}$-Estern und deren Gemischen ausgewählt ist, mindestens eines bei Raumtemperatur flüssigen, in dem flüchtigen Lösungsmittel löslichen oder dispergierbaren, nicht flüchtigen Silicons und mindestens eines in dem flüchtigen Lösungsmittel löslichen und mit dem nicht flüchtigen Silicon inkompatiblen, nicht flüchtigen Kohlenwasserstofföls, wobei das nicht flüchtige Kohlenwasserstofföl die Löslichkeitsparameter 16,40 $(J/cm^3)^{1/2} \leq \delta_D \leq 19{,}00\ (J/cm^3)^{1/2}$ und 2,00 $(J/cm^3)^{1/2} \leq \delta_a \leq 9{,}08\ (J/cm^3)^{1/2}$ und eine Molmasse über 600 g/mol aufweist und wobei die Zusammensetzung sich nicht überträgt und/oder glänzt.

**26.** Nicht therapeutisches Verfahren zur kosmetischen Pflege oder zum Schminken der Lippen oder der Haut, das darin besteht, auf die Lippen oder die Haut eine kosmetische Zusammensetzung nach einem der Ansprüche 1 bis 24 aufzutragen.

**27.** Kosmetisches Verfahren, um den Transfer einer kosmetischen Zusammensetzung zum Schminken oder zur Pflege der Haut oder der Lippen auf einen Träger, der sich von der Haut oder den Lippen unterscheidet, zu vermindern oder sogar zu verhindern, wobei die Zusammensetzung mindestens einen Bestandteil enthält, der unter den kosmetischen Wirkstoffen und Farbmitteln ausgewählt ist, das darin besteht, in die Zusammensetzung ein System einzuarbeiten, das mindestens einen flüchtigen Kohlenwasserstoff als Lösungsmittel, der unter den verzweigten $C_{8-16}$-Alkanen, den verzweigten $C_{8-14}$-Estern und deren Gemischen ausgewählt ist, mindestens ein bei Raumtemperatur flüssiges, in dem flüchtigen Kohlenwasserstoff lösliches oder dispergierbares, nicht flüchtiges Silicon und mindestens ein in dem flüchtigen Lösungsmittel lösliches und mit dem nicht flüchtigen Silicon inkompatibles, nicht flüchtiges Kohlenwasserstofföl enthält, wobei das nicht flüchtige Kohlenwasserstofföl die Löslichkeitsparameter 6,40 $(J/cm^3)^{1/2} \leq \delta_D \leq 19{,}00\ (J/cm^3)^{1/2}$ und 2,00 $(J/cm^3)^{1/2} \leq \delta_a \leq 9{,}08\ (J/cm^3)^{1/2}$ aufweist und eine Molmasse über 600 g/mol besitzt.

## Claims

**1.** Care or make-up composition for keratin materials, comprising at least one volatile hydrocarbon-based solvent chosen from branched $C_8$-$C_{16}$ alkanes and branched $C_8$-$C_{14}$ esters, and mixtures thereof, at least one non-volatile silicone compound which is liquid at room temperature and soluble or dispersible in the volatile hydrocarbon-based solvent, and at least one non-volatile hydrocarbon-based oil which is soluble in the volatile solvent and incompatible with the non-volatile silicone compound, the non-volatile hydrocarbon-based oil having solubility parameters such that 16.40 $(J/cm^3)^{1/2} \leq \delta_D \leq 19.00\ (J/cm^3)^{1/2}$ and 2.00 $(J/cm^3)^{1/2} \leq \delta_a \leq 9.08\ (J/cm^3)^{1/2}$ and having a molar mass of greater than 600 g/mol.

**2.** Composition according to Claim 1, **characterized in that** the hydrocarbon-based oil has solubility parameters such that 16.70 $(J/cm^3)^{1/2} \leq \delta_D \leq 18.50\ (J/cm^3)^{1/2}$.

**3.** Composition according to Claim 2, **characterized in that** the hydrocarbon-based oil has solubility parameters such that 4.00 $(J/cm^3)^{1/2} \leq \delta_a \leq 9.08\ (J/cm^3)^{1/2}$.

**4.** Composition according to one of the preceding claims, **characterized in that** the hydrocarbon-based oil has a chemical structure comprising at least one nonionic polar group chosen from -COOH; -OH; $-PO_4$; NHR; $NR_1R_2$ with $R_1$ and $R_2$ optionally forming a ring and representing a linear or branched $C_1$ to $C_{20}$ alkyl or alkoxy radical, or

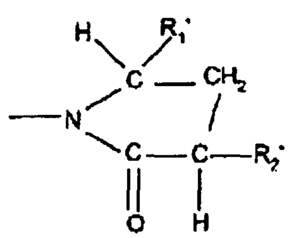

with $R_1'$ and $R_2'$ which may be equal to H or to a linear or branched $C_1$ to $C_{20}$ alkyl or alkoxy chain.

**5.** Composition according to one of the preceding claims, **characterized in that** the hydrocarbon-based oil has a chemical structure comprising at least two nonionic polar groups chosen from -COOH; -OH; $-PO_4$; or

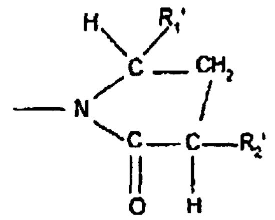

with $R_1'$ and $R_2'$ which may be equal to H or to a linear or branched $C_1$ to $C_{20}$ alkyl or alkoxy chain.

**6.** Composition according to one of the preceding claims, **characterized in that** the non-volatile hydrocarbon-based oil is chosen from diisostearyl malate, polyol monoesters and polyesters and poly(12-hydroxy)stearic acids, and mixtures thereof.

**7.** Composition according to one of the preceding claims, **characterized in that** the non-volatile hydrocarbon-based oil is chosen from diglyceryl diisostearate, diglyceryl triisostearate and poly(12-hydroxy)stearic acids, and mixtures thereof.

**8.** Composition according to one of the preceding claims, **characterized in that** the non-volatile hydrocarbon-based oil is present at a content by mass ranging from 2.5% to 40%, preferably from 4% to 30% and better still from 4% to 15%.

**9.** Composition according to one of the preceding claims, in which the non-volatile silicone compound has a viscosity chosen within the range from 5 to 10 000 cSt and preferably from 10 to 5 000 cSt (0.1-50 $cm^2/s$).

**10.** Composition according to one of the preceding claims, in which the non-volatile silicone compound is chosen from non-volatile polydimethylsiloxanes (PDMSs); polydimethylsiloxanes comprising alkyl, alkoxy or phenyl groups that are pendent or at the end of a silicone chain, these groups containing from 2 to 24 carbon atoms; phenyl trimethicones, phenyl dimethicones, phenyl trimethylsiloxydiphenylsiloxanes, diphenyl dimethicones, diphenyl methyldiphenyltrisiloxanes and 2-phenylethyl trimethylsiloxysilicates; fluorosilicones comprising a fluoro group which is pendent or at the end of a silicone chain and containing from 1 to 12 carbon atoms, all or some of the hydrogen atoms of which are substituted with fluorine atoms; silicone resins, and mixtures thereof.

**11.** Composition according to one of the preceding claims, **characterized in that** the non-volatile silicone compound represents a content by mass of from 0.5% to 90%, preferably from 5% to 60% and better still from 10% to 50%.

**12.** Composition according to one of the preceding claims, in which at least one ingredient chosen from cosmetic active agents, dermatological active agents and dyestuffs is provided.

**13.** Composition according to one of the preceding claims, in which the volatile solvent is chosen from $C_8$-$C_{16}$ isoparaffins, isododecane and isohexadecane, and mixtures thereof.

**14.** Composition according to one of the preceding claims, in which the volatile solvent is chosen from isododecane and isohexadecane, and mixtures thereof.

**15.** Composition according to one of the preceding claims, in which the volatile solvent represents a content by mass of from 5% to 90%, preferably from 10% to 60% and better still from 20% to 50%.

**16.** Composition according to one of the preceding claims, in which the ratio by mass of non-volatile silicone compound relative to the non-volatile hydrocarbon-based oil is greater than or equal to 1.

**17.** Composition according to one of the preceding claims, also comprising at least one fatty substance other than the non-volatile silicone compound, the volatile hydrocarbon-based solvent and the non-volatile hydrocarbon-based oil, chosen from waxes, gums and fatty substances that are pasty at room temperature, and oils, and mixtures thereof.

**18.** Composition according to Claim 12, in which the dyestuffs comprise at least one pulverulent compound chosen from fillers, pigments and nacres, and mixtures thereof.

**19.** Composition according to Claim 18, **characterized in that** the pulverulent compound represents up to 50% of the total weight of the composition.

**20.** Composition according to one of the preceding claims, which is in the form of a stick or tube, in the form of a soft paste, in the form of a dish, an oily gel, an oily liquid, a vesicular dispersion containing ionic and/or nonionic lipids, or a water-in-oil or oil-in-water emulsion.

**21.** Composition according to one of the preceding claims, which is in anhydrous form.

**22.** Composition according to one of the preceding claims, which is in the form of a foundation, a blusher, an eyeshadow, a lipstick, a care base or care balm for the lips, a concealer product, an eyeliner or a mascara.

**23.** Lipstick comprising at least one volatile hydrocarbon-based solvent chosen from branched $C_8$-$C_{16}$ alkanes and branched $C_8$-$C_{14}$ esters, and mixtures thereof, at least one non-volatile silicone compound which is liquid at room temperature and soluble or dispersible in the volatile hydrocarbon-based solvent. and at least one non-volatile hydrocarbon-based oil which is soluble in the volatile solvent and incompatible with the non-volatile silicone compound, the non-volatile hydrocarbon-based oil having solubility parameters such that 16.40 $(J/cm^3)^{1/2} \leq \delta_D \leq$ 19.00 $(J/cm^3)^{1/2}$ and 2.00 $(J/cm^3)^{1/2} \leq \delta_a \leq$ 9.08$(J/cm^3)^{1/2}$ and having a molar mass of greater than 600 g/mol.

**24.** Use, in a cosmetic composition or for the manufacture of a composition for topical application, of a combination of at least one volatile hydrocarbon based solvent chosen from branched $C_8$-$C_{16}$ alkanes and branched $C_8$-$C_{14}$ esters, and mixtures thereof, of at least one non-volatile silicone compound which is liquid at room temperature and soluble or dispersible in the volatile solvent and of at least one non-volatile hydrocarbon-based oil which is soluble in the volatile solvent and incompatible with the non-volatile silicone compound, the non-volatile hydrocarbon-based oil having solubility parameters such that 16.40 $(J/cm^3)^{1/2} \leq \delta_D \leq$ 19.00 $(J/cm^3)^{1/2}$ and 2.00 $(J/cm^3)^{1/2}$ $\leq \delta_a \leq$ 9.08 $(J/cm^3)^{1/2}$ and having a molar mass of greater than 600 g/mol, as an agent for reducing or even preventing altogether the transfer of a film of composition deposited on the skin and/or the lips of a human being to a support placed in contact with the film and/or for preserving its gloss.

**25.** Use, in a cosmetic composition or for the manufacture of a composition for topical application, of a combination of at least one volatile hydrocarbon-based solvent chosen from branched $C_8$-$C_{16}$ alkanes and branched $C_8$-$C_{14}$ esters, and mixtures thereof, of at least one non-volatile silicone compound which is liquid at room temperature and soluble or dispersible in the volatile solvent, and of at least one non-volatile hydrocarbon-based oil which is soluble in the volatile solvent and incompatible with the non-volatile silicone compound, the non-volatile hydrocarbon-based oil having solubility parameters such that 16.40 $(J/cm^3)^{1/2} \leq \delta_D \leq$ 19.00 $(J/cm^3)^{1/2}$ and 2.00 $(J/cm^3)^{1/2}$ $\leq \delta_a \leq$ 9.08 $(J/cm^3)^{1/2}$ and having a molar mass of greater than 600 g/mol, the said composition being transfer-resistant and/or glossy.

**26.** Non-therapeutic cosmetic care process or make-up process for the lips or the skin, which consists in applying a cosmetic composition as defined in Claims 1 to 24 to the lips or the skin, respectively.

**27.** Cosmetic process for limiting, or even preventing altogether, the transfer of a cosmetic care or make-up composition for the skin or the lips onto a support other than the skin or the lips, containing at least one ingredient chosen from cosmetic active agents and dyestuffs, which consists in introducing into the composition a system containing at least one volatile hydrocarbon-based solvent chosen from branched $C_8$-$C_{16}$ alkanes and branched $C_8$-$C_{14}$ esters, and mixtures thereof, at least one non-volatile silicone compound which is liquid at room temperature and soluble or dispersible in the volatile solvent, and at least one non-volatile hydrocarbon-based oil which is soluble in the volatile solvent and incompatible with the non-volatile silicone compound, the hydrocarbon-based oil having solubility parameters such that 16.40 $(J/cm^3)^{1/2} \leq \delta_D \leq$ 19.00 $(J/cm^3)^{1/2}$ and 2.00 $(J/cm^3)^{1/2} \leq \delta_a \leq$ 9.08 $(J/cm^3)^{1/2}$ and having a molar mass of greater than 600 g/mol.